(19)

Europäisches
Patentamt
European
Patent Office
Office européen
des brevets

(11) **EP 1 744 180 A1**

(12) **EUROPEAN PATENT APPLICATION**

(43) Date of publication:
**17.01.2007 Bulletin 2007/03**

(51) Int Cl.:
**G01S 15/89** (2006.01)   **G01S 7/52** (2006.01)

(21) Application number: **06003001.2**

(22) Date of filing: **15.02.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **15.07.2005  KR 2005064293**

(71) Applicant: **MEDISON CO., LTD.**
**Kangwon-do 250-870 (KR)**

(72) Inventors:
• **Lee, Seung Woo**
  **Gangnam-gu**
  **Seoul 135-955 (KR)**
• **Bae, Moo Ho**
  **Songpa-gu**
  **Seoul 138-240 (KR)**

(74) Representative: **Lorenz, Werner**
  **Lorenz & Kollegen**
  **Patent- und Rechtsanwaltskanzlei**
  **Alte Ulmer Strasse 2**
  **89522 Heidenheim (DE)**

(54) **Ultrasound system for reconstructing an image with the use of additional information**

(57)     There is provided an ultrasound system for constructing or reconstructing an image with the use of pre-stored data and additional signals. The ultrasound system includes: a probe including a number of transducers for transmitting ultrasound transmission signals to a target object, receiving echo signals reflected from the target object and transducing the echo signals into electrical signals; an analog-to-digital conversion unit for converting the electrical signals into digital data; a transducer information collecting unit for collecting information on spatial states of the transducers at the time of receiving the echo signals; a beam-former for forming reception beams based on the converted digital data and the collected information; and an ultrasound image processing unit for reconstructing an ultrasound image based on the reception beams.

**Fig. 3**

**Description**

The present invention generally relates to an ultrasound system, and more particularly to an ultrasound system for reconstructing a 2-dimensional (2-D) or 3-dimensional (3-D) image through the use of pre-stored image data and additional information.

A diagnostic ultrasound system is now widely used to inspect an internal state of a human body. The ultrasound system may obtain an image of a single layer or a blood flow of a soft tissue without using an invasive needle. This is typically done through the process of radiating an ultrasound signal to a desired portion in the human body from a body surface of a target object to be diagnosed, receiving the reflected ultrasound signal, and processing the received ultrasound signal (the ultrasound echo signal). Compared to other medical imaging systems (e.g., X-ray diagnostic system, X-ray Computerized Tomography scanner, Magnetic Resonance Imaging system, nuclear medicine diagnostic system, etc.), the ultrasound diagnostic system is relatively small in size and inexpensive, capable of displaying images in real-time, highly safe from exposure to X-ray radiation, etc. Due to such advantages, the ultrasound diagnostic system is extensively employed for diagnosing the heart, abdomen and urinary organs, especially in the fields of obstetrics and gynecology, etc.

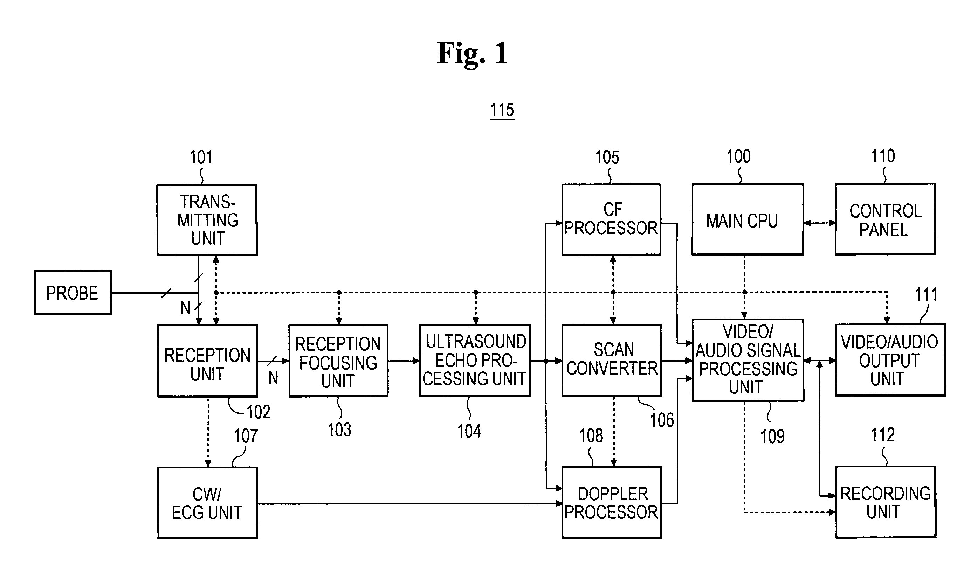

Fig. 1 shows a functional block diagram of a conventional ultrasound system. As shown in Fig. 1, a conventional ultrasound system 115 generally includes a main CPU 100, a transmitting unit 101, a reception unit 102, a receive-focusing unit 103, an ultrasound echo processing unit 104, a Color Flow (CF) processor 105, a scan converter 106, a Continuous Wave/ElectroCardioGram (CW/ECG) unit 107, a Doppler processor 108, a video/audio signal processing unit 109, a control panel 110, a video/audio output unit 111, a recording unit 112 and a probe having a plurality of transducers (not shown).

A user inputs commands through the control panel 110. The CPU 100 responds to the user's commands to control the entire ultrasound system 115. The transmitting unit 101 delivers transmission pulses to the probe. The transmission pulses are transduced into ultrasound signals at a multiplicity of transducers arranged in an array form in the probe, and are transmitted to a target object where the transmission pulses are reflected. The reception unit 102 receives the signals reflected from the target object (a human body) via the transducers. The reception unit 102 then performs front-end amplification, TGC (Time Gain Compensation), and filtering for anti-aliasing upon the reflected signals. The receive-focusing unit 103 performs dynamic focusing for each image point on signals outputted from the reception unit 102 to thereby maximize the resolution of an ultrasound image. The CW/ECG unit 107 analyzes the received signals from the reception unit 102 to generate electrocardiogram (ECG) waveform bio-information. The ultrasound echo processing unit 104 processes high frequency signals and base band signals in the signals focused at the receive-focusing unit 103, and outputs the resulting output signals.

The CF processor 105 and the scan converter 106 receive the output signals from the ultrasound echo processing unit 104, and create a 2-D CF image and a B-mode image, respectively. The Doppler processor 108 forms a spectral Doppler waveform based on the output signals of the ultrasound echo processing unit 104 and the output signals of the CW/ECG unit 107. The video/audio signal processing unit 109 processes video/audio signals outputted from the CF processor 105, the scan converter 106 and the Doppler processor 108. The processed result is provided to the video/audio output unit 111 and the recording unit 112 for the user's observation and storage, respectively. In this way, an ultrasound image for a desired target object can be obtained through the use of the ultrasound system.

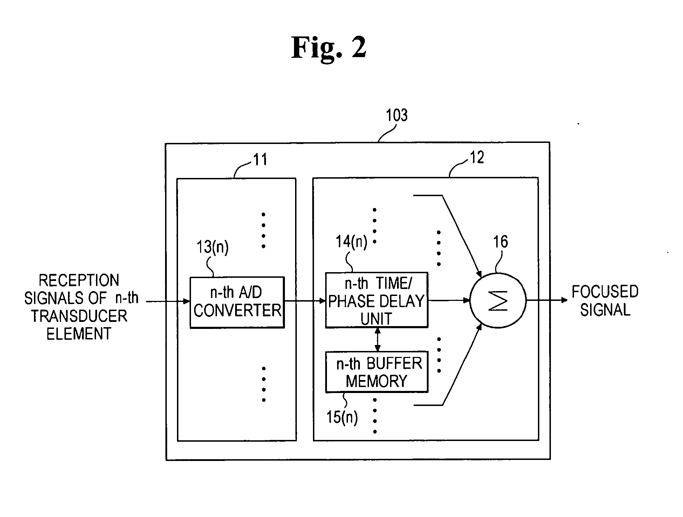

Fig. 2 is a diagram showing a configuration of the receive-focusing unit 103. The receive-focusing unit 103 may include an A/D conversion unit 11 and a focusing unit 12. The A/D conversion unit 11 includes a number of A/D converters, each being coupled to the respective transducer in the probe. The focusing unit 12 includes a number of time/phase delay units, a number of associated buffer memories, and an adder 16. Each of the time/phase delay units is respectively coupled to one of the A/D converters.

The A/D conversion unit 11 converts the signals received from the N number of transducers into digital signals. The focusing unit 12 focuses the digital output signals from the A/D conversion unit 11 to provide the focused signals. For example, the n-th A/D converter 13(n) in the A/D conversion unit 11 samples the signals received from the n-th transducer. The n-th time/phase delay unit 14(n) in the focusing unit 12 provides a time delay or a phase delay on the sampled signals to output the delayed signals. The n-th time/phase delay unit 14(n) may utilize n-th buffer memory 15(n) of a short length in order to temporarily store the output signals of the n-th A/D converter 13(n). For the buffer memory, a FIFO (first-in first-out) type memory or 2-port memory may preferably be used.

For example, n-th A/D converter 13(n) in the A/D conversion unit 11 samples the signals received from n-th transducer out of the N number of transducers. Further, n-th time/phase delay unit 14(n) in the focusing unit 12 performs a time delay or a phase delay on output signals of the A/D converter 13(n), and provides the result signals to the adder 16. The n-th time/phase delay unit 14(n) utilizes n-th buffer memory 15(n) of a short length in order to temporarily store the output signals of the n-th A/D converter 13(n). As the buffer memory, the FIFO (first-in first-out) type memory or 2-port memory is mainly used.

Since the delayed signals corresponding to different ultrasound image points are mostly different from each other, output signals of the A/D converters stored in the aforementioned buffer memories are changed continually. Accordingly, the output signals of the A/D converters stored in the buffer memories are all removed after a focusing process. Therefore, they cannot be reused, which poses to be a problem.

Further, the signals received by the transducers cannot be precisely focused due to several types of waveform distortion phenomena that occur when the ultrasounds move through a human body. Therefore, it is impossible to actually obtain an ultrasound image having a theoretically obtainable resolution.

It is, therefore, an object of the present invention to provide an ultrasound system that can improve the resolution of an image by storing image data, which are received from transducers within a probe, in a memory in order to reconstruct a 2-D or 3-D image based on the stored image data and additional information.

In accordance with a preferred embodiment of the present invention, in order to achieve the above-mentioned object, there is provided an ultrasound system including: a probe including a number of transducers for transmitting ultrasound transmission signals to a target object, receiving echo signals reflected from the target object and transducing the echo signals into electrical signals; an analog-to-digital conversion unit for converting the electrical signals into digital data; a transducer information collecting unit for collecting information on spatial states of the transducers at the time of receiving the echo signals; a beam-former for forming reception beams based on the converted digital data and the collected information; and an ultrasound image processing unit for reconstructing an ultrasound image based on the reception beams.

In accordance with another preferred embodiment of the present invention, there is provided an ultrasound system including: a probe including a number of transducers for transmitting ultrasound transmission signals to a target object, receiving echo signals reflected from the target object and transducing the echo signals into electrical signals; an analog-to-digital conversion unit for converting the electrical signals into digital data; a spatial information generating unit for generating spatial information on the transducers according to a movement tendency of the probe, said generated spatial information being changed in time; a beam-former for forming reception beams based on the converted digital data and the change in the generated spatial information; and an ultrasound image processing unit for reconstructing an ultrasound image based on the reception beams.

In accordance with yet another preferred embodiment of the present invention, there is provided an ultrasound system for reconstructing an ultrasound image corresponding to a portion selected by a user out of a preformed ultrasound image, the system including: a probe including a number of transducers for transmitting ultrasound transmission signals to a target object, receiving echo signals reflected from the target object and transducing the echo signals into electrical signals; a transducer information collecting unit for collecting information on spatial states of the transducers at the time of receiving the echo signals; a receive-focusing unit including an analog-to-digital conversion unit for converting the electrical signals into digital data and a memory for storing the converted digital data to accumulate at least one frame of data for at least a part of the target object therein, said receive-focusing unit being configured to focus the converted digital data in consideration of the collected information, said receive-focusing unit being operable to retrieve and focus the stored digital data corresponding to the selected portion in consideration of the collected information; and an ultrasound image processing unit for forming an ultrasound image based on the focused digital data.

The above and other objects and features in accordance with the present invention will become apparent from the following descriptions of preferred embodiments given in conjunction with the accompanying drawings, in which:

Fig. 1 shows a functional block diagram of a conventional ultrasound system;

Fig. 2 is a diagram showing a configuration of a receive-focusing unit shown in Fig. 1;

Fig. 3 shows a block diagram of an ultrasound system constructed in accordance with a first preferred embodiment of the present invention;

Figs. 4A to 4C, 5A and 5B are drawings for explaining a need to reflect spatial information relating to a movement of a probe;

Figs. 6A to 6C show a variation in location of overlap between beams according to a movement of a probe;

Fig. 7 shows a block diagram of an ultrasound system constructed in accordance with a second preferred embodiment of the present invention;

Fig. 8 shows a detailed block diagram of a receive-focusing unit;

Fig. 9 shows a detailed block diagram of a memory controller in a receive-focusing unit;

Fig. 10 is a diagram showing a memory in a receive-focusing unit;

Fig. 11 shows a block diagram of a receive-focusing unit in an ultrasound system constructed in accordance with another embodiment of the present invention;

Fig. 12 shows a block diagram of a receive-focusing unit in an ultrasound system constructed in accordance with yet another embodiment of the present invention; and

Fig. 13 shows a block diagram of an ultrasound system constructed in accordance with a third preferred embodiment of the present invention.

Hereinafter, preferred embodiments of the present invention will be described with reference to the accompanying drawings.

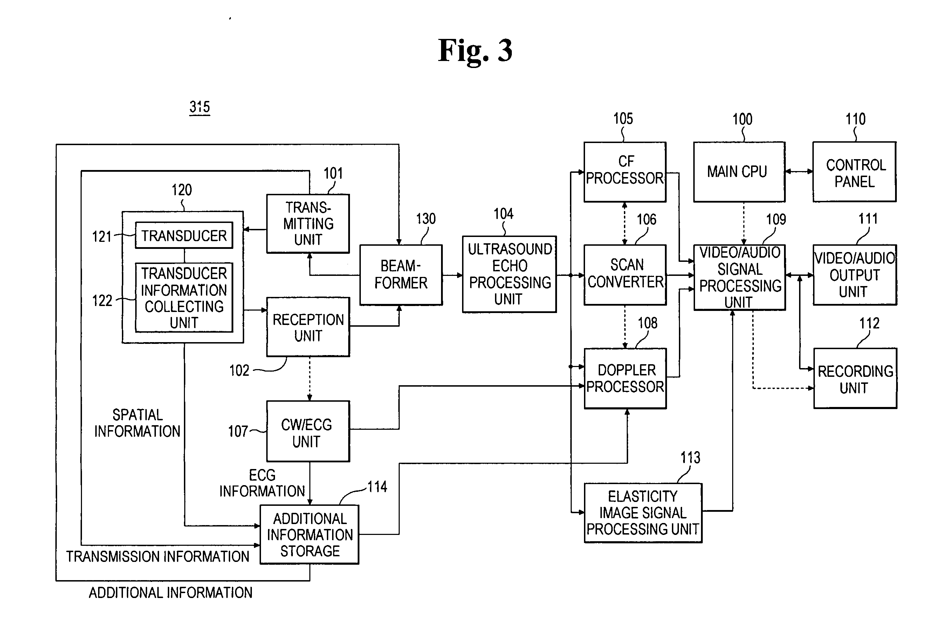

Fig. 3 shows a block diagram of an ultrasound system 315 constructed in accordance with a first preferred embodiment of the present invention. The ultrasound system 315 constructed in accordance with the present invention generally includes a main CPU 100, a transmitting unit 101, a reception unit 102, a beam-former 130, a ultrasound echo processing unit 104, a Color Flow (CF) processor 105, a scan converter 106, a Continuous Wave/ElectroCardioGram (CW/ECG) unit 107, a Doppler processor 108, a video/audio signal processing unit 109, a control panel 110, a video/audio output unit 111, a recording unit 112, an elasticity image signal processing unit 113, an additional information storage 114 and a probe 120.

In the probe 120, transducers 121 and a transducer information collecting unit (transducer sensor) 122 are generally provided. Further, a pressure sensor (not shown) may preferably be provided in the probe 120. Although Fig. 3 shows that the transducer information collecting unit 122 is embodied in the probe 120, it should be recognized herein that such unit 122 may be excluded from the probe 120.

The transducer information collecting unit 122 collects spatial information for the transducers (information on spatial changes of the transducers) as first additional information, as well as time information. Spatial information for a transducer generally includes information regarding location and direction of the transducer at the time of obtaining an echo signal. The location and direction of the transducer may preferably be positional information relative to a target object or other transducers. The time information for the transducer is the time of obtaining the echo signal. The transducer information collecting unit 122 may be embodied in various types of position sensors, such as an optical position sensor, a position sensor utilizing an ultrasound in the air, a position sensor utilizing a magnetic field, and the like. By coupling at least one type of position sensor with the probe, that is, by coupling the position sensor with the probe in a built-in way or a removable way, the location and direction of the entire probe can be obtained. The location of each transducer in the probe can be obtained with the use of three position sensors.

The CW/ECG unit 107 analyzes RF reception signals inputted through the reception unit 102 to generate electrocardiogram (ECG) waveform bio-information, which is the second additional information.

The first additional information and the second additional information are stored in the additional information storage 114. Further, the additional information storage 114 receives the third additional information from the transmitting unit 101 and stores it. The third additional information may include information regarding the type of transmission signals (e.g., coded Tx, pulse or the like) transmitted to obtain the echo signals, as well as information regarding the beam-forming conditions such as a focal point location, an aperture size, an intensity and the like, and information regarding whether or not to use a sound field.

The beam-former 130 generates more than one transmission pulses having different delay values and delivers them toward the transmitting unit 101. The transmitting unit 101 amplifies the transmission pulses and transmits them to the probe. Then, the transmission pulse signals are transduced into ultrasound signals by transducers generally included in the probe and the ultrasound signals are transmitted to a target object. Echo signals reflected from the target object are transduced into electrical signals (RF reception signals) by the transducers in the probe so as to be transmitted to the reception unit 102. The beam-former 130 receives the RF signals and forms reception beams. Herein, the beam-former 130 forms the reception beams with reference to additional information stored in the additional information storage 114.

The ultrasound echo processing unit 104 processes high frequency signals and base band signals in the reception beams formed by the beam-former 130. The CF processor 105 creates a 2-D CF image based on the output signals of the ultrasound echo processing unit 104. The scan converter 106 creates a B-mode image based on the output signals of the ultrasound echo processing unit 104. The elasticity image signal processing unit 113 outputs an elasticity image signal, which represents change of pressure given to the probe, based on signals inputted from the ultrasound echo signal processing unit 104. Meanwhile, the probe may preferably further include a pressure sensor. In case of utilizing information obtained from the pressure sensor mounted on the probe, an improved elasticity image signal can be obtained. The Doppler processor 108 forms a spectral Doppler waveform based on the signals and information inputted from the ultrasound echo processing unit 104 and the CW/ECG unit 107. The video/audio signal processing unit 109 processes, under the control of the main CPU 100, video/audio signals and the like inputted from the CF processor 105, the scan converter 106, the Doppler processor 108 and the elasticity image signal processing unit 113. The video/audio signal processing unit 109 then provides them toward the video/audio output unit 111 and the recording unit 112. With the use of such a digital ultrasound system, an ultrasound image for a desired target object is obtained.

The ultrasound system constructed in accordance with the present invention can improve the quality of an ultrasound image by forming reception beams based on additional information. For example, it can reduce motion blur by calculating focusing delays, wherein a probe movement in a plane is reflected based on spatial information of transducers, and forming the image in consideration of the calculated result. Further, by forming the reception beams in consideration of position information of each transducer such as an angle between it and a focusing point or the like, it can diminish a speckle pattern as well as providing a better view of a portion behind an obstacle that forms a shadow.

Moreover, even while the probe is moving along an elevation direction, it can catch the movement of the probe accurately. A synthetic focusing can be done along the elevation direction. Accordingly, an elevation focusing, which was practicable only with the use of 2-D array, becomes practicable even when using 1-D array probes.

Further, when the additional information obtained from the ECG waveform is reflected, the image can be formed in consideration of a tissue motion along a heartbeat period, which makes it possible to reduce an image blur and to predict a tissue motion.

Meanwhile, it may be necessary to move a probe along a predetermined path with a regular speed on a surface of a target object so as to obtain more accurate additional information, and more particularly to obtain spatial information of transducers. For this purpose, the ultrasound system constructed in accordance with the present invention may preferably further include a probe movement device. The probe movement device may preferably be embodied to make a movement or a stop with the use of robot arms or the like. Alternatively, an automatic movement device may be built in the probe, or a removable movement device may be coupled to a surface of the probe. Moreover, the aforementioned position sensors and the probe movement device can be embodied compositively.

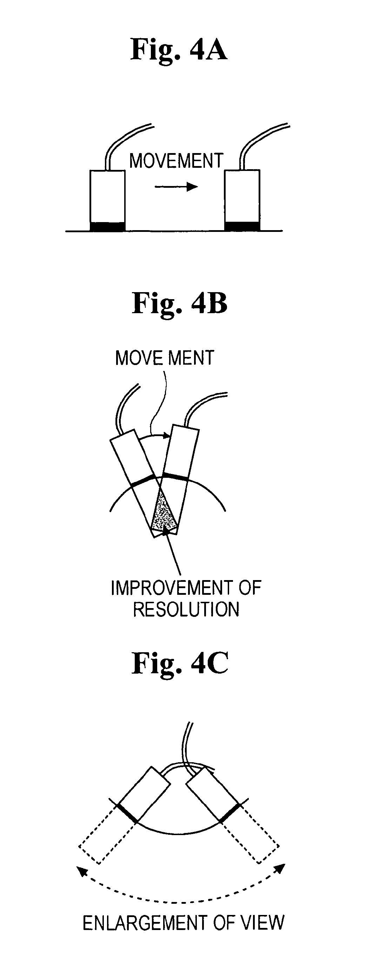

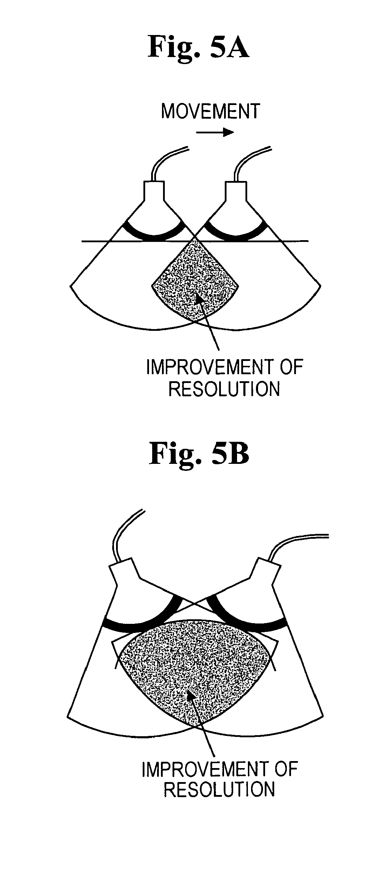

In case a probe having transducers in a linear array or a probe having transducers in a convex array moves linearly in a lateral direction as shown in Fig. 4A or Fig. 5A, the positions of the transducers change with time due to the movement. The present invention catches the change through using the spatial information and forms receptions beams in consideration of the spatial information. In such a case, the reception beams are formed through adjusting and changing focusing delays, which were used when the probe movement was not considered.

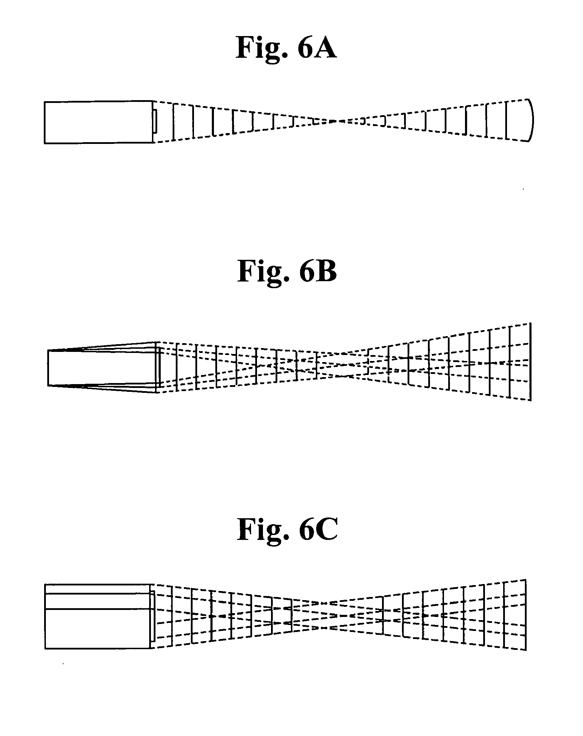

In case a probe having transducers in a linear array moves on a curved surface as shown in Figs. 4B and 4C, or in case a probe having transducers in a convex array moves on a curved surface as shown in Fig. 5B, the angles between the respective transducers and a focusing point are different from each other. For example, in case of a probe having a beam profile as shown in Fig. 6A in an elevation-axial plane, a curve (rotation) movement and a linear movement along the elevation direction result in different locations of overlap between the beams (shown in Figs. 6B and 6C). The present invention can reflect such a difference also as additional information when forming reception beams, thereby improving the quality of an ultrasound image.

The ultrasound system constructed in accordance with the aforementioned embodiment of the present invention is characterized by forming reception beams through using additional information collected by the transducer information collecting unit and the like. The additional information can be also utilized for reconstructing a 2-D or 3-D image.

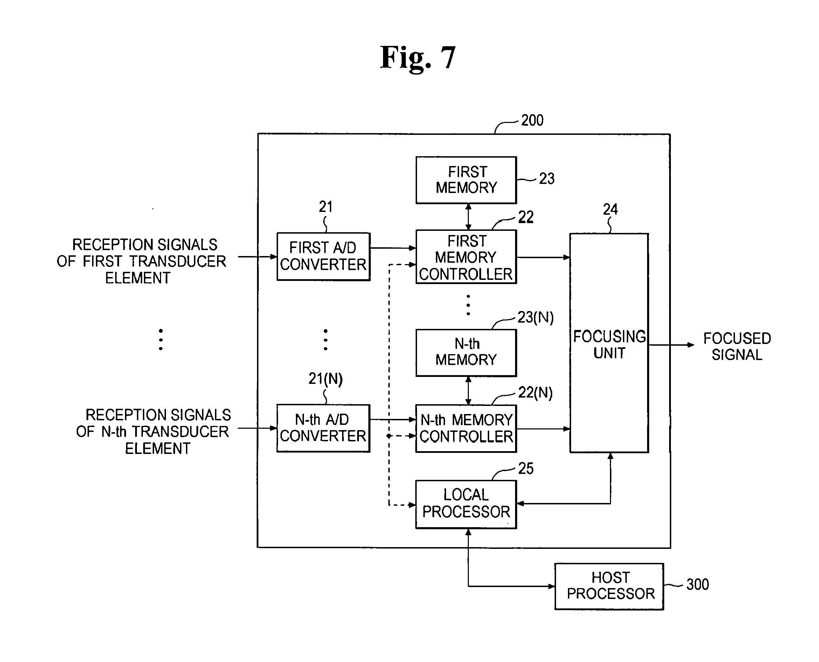

Fig. 7 shows a block diagram of an ultrasound system 715, which is constructed in accordance with a second preferred embodiment of the present invention. The ultrasound system 715 constructed in accordance with the present invention generally includes a main CPU (control processing unit) 100, a transmitting unit 101, a reception unit 102, a receive-focusing unit 200, an ultrasound echo processing unit 104, a Color Flow (CF) processor 105, a scan converter 106, a Continuous Wave/ElectroCardioGram (CW/ECG) unit 107, a Doppler processor 108, a video/audio signal processing unit 109, a control panel 110, a video/audio output unit 111, a recording unit 112, an elasticity image signal processing unit 113, an additional information storage 114 and a probe 120. Configurations and functions in the organization, which differ from those in Fig. 3, will be described hereinafter.

The receive-focusing unit 200 receives additional information, which is stored in the additional information storage 114, and RF reception signals from the reception unit 102. The receive-focusing unit 200 then performs a synthetic focusing with the inputted reception signals and additional information. That is, it reflects at least the first additional information in focusing the reception signals. A more detailed organization and functions of the receive-focusing unit 200 will be described later.

The ultrasound echo processing unit 104 processes high frequency signals and base band signals in the reception signals focused by the receive-focusing unit 200. The main CPU 100 controls a host processor 300 and the video/audio signal processing unit 109. The host processor 300 controls the receive-focusing unit 200, the reception unit 102, the ultrasound echo processing unit 104, the CF processor 105, the scan converter 106, the Doppler processor 108, and the video/audio output unit 111. The functions of the host processor 300 can also be implemented in the main CPU 100.

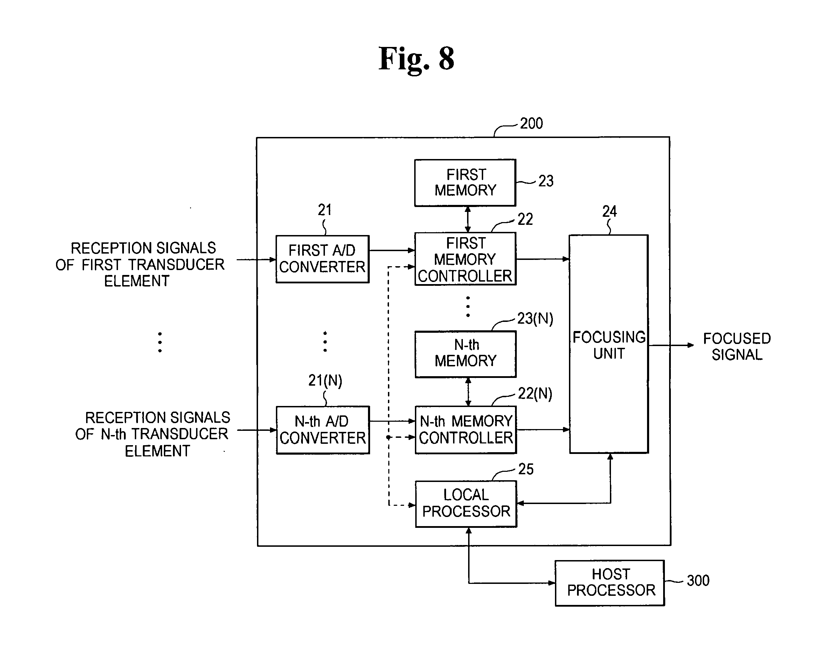

Hereinafter, the organization and operations of the receive-focusing unit will be discussed in more detail with reference to Figs. 8 to 12.

Fig. 8 shows a detailed block diagram of the receive-focusing unit 200 in accordance with an embodiment of the present invention. The receive-focusing unit 200 generally includes: A/D converters 21~21(N) for converting signals received from the respective transducers; memory controllers 22~22(N) for receiving the transduced reception signals and additional information from the respective A/D converters 21~21(N) and the host processor 300; memories 23~23(N) for storing at least one frame of the reception signals, corresponding to a portion or whole of a target object, and additional information, which were inputted via the respective memory controllers 22~22(N); a focusing unit 24 for, when there is a request for reconstruction of a portion of a preformed ultrasound image, receiving the reception signals and additional information stored in the memory 23~23(N) via the memory controller 22~22(N), and performs the focusing; and a local processor 25 for analyzing the reception signals and additional information inputted through the memory controller 22~22(N), and controlling the focusing unit 24 based on the analysis result to obtain an optimal ultrasound image.

The local processor 25 is controlled by the host processor 300. The memory controllers 22~22(N) and the focusing unit 24 may be directly controlled by the host processor 300. In such a case, the local processor 25 can be omitted. Further, the local processor 25 can be prepared plurally, each local processor being coupled to each memory controller 22~22(N). If a portion of an image is selected by a user through the control panel 110 during a real-time ultrasound image output of the ultrasound system or after pausing the ultrasound image output, the memory controllers 22~22(N) read, under the control of the local processor 25, the reception signals corresponding to the selected image and the additional information from the memory 23~23(N), and transmits them to the focusing unit 24.

Hereinafter, the operations of the receive-focusing unit 200 having the aforementioned configuration will be described.

The A/D converters 21~21(N) in the receive-focusing unit 200 transduce reception signals inputted from the N number of transducers and provide them to the memory controller 22~22(N). The memory controllers 22~22(N) transmit the reception signals, inputted from the A/D converters 21~21(N), toward the memories 23~23(N) along paths specified by the control of the local processor 25. The memory controllers 22~22(N) then receive additional information through the local processor 25 coupled to the host processor 300 and transmit it toward the focusing unit 24.

As mentioned above, the reception signals are at once stored in the memories 23~23(N) and focused in the focusing unit 24. Further, the memory controllers 22~22(N) read the data stored in the memories 23~23(N) and transmit it to the focusing unit 24 and the local processor 25 under the control of the local processor 25.

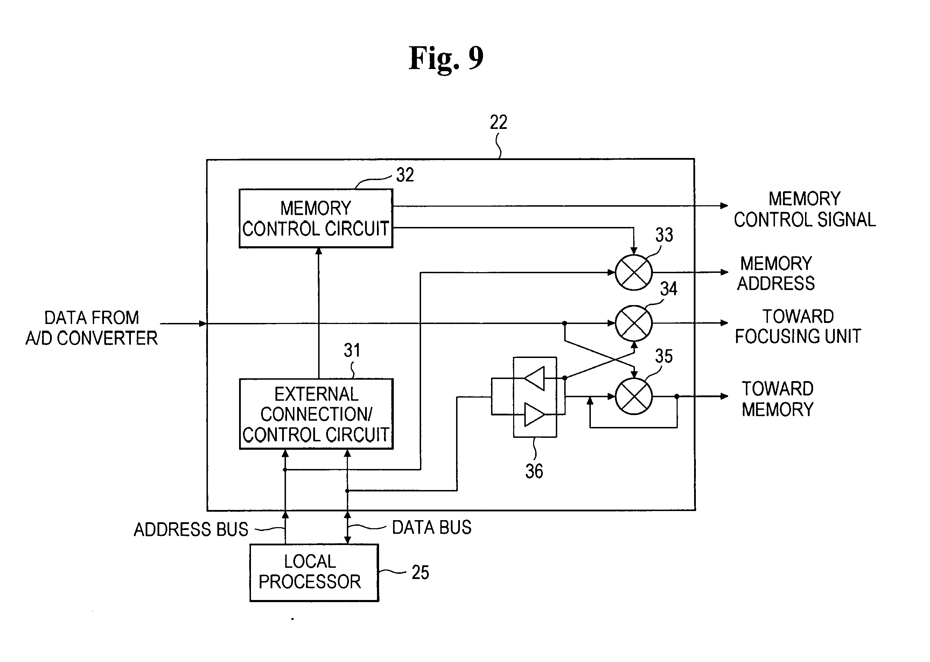

Hereinafter, the configurations and operations of the memory controller 22 (among a plurality of memory controllers 22~22(N)) will be described with reference to Fig. 9.

The memory controller 22 generally includes: an external connection/control circuit 31 connected to the local processor 25; a memory control circuit 32 for generating a memory control signal and a memory address to read/write data from/in the memory 23 according to a request from the local processor 25 under the control of the external connection/control circuit 31; a multiplexer 33 for receiving the memory address from the memory control circuit 32 and outputting it; multiplexers 34 and 35 for receiving and transmitting signals from the A/D converter 21 to the focusing unit 24 and the memory 23; and a buffer 36 for temporarily storing the data stored in the memory 23 (reception signals) and data inputted from the local processor 25. The signal data stored in the buffer 36 is transmitted to the local processor 25, or to the focusing unit 24 via the multiplexer 34.

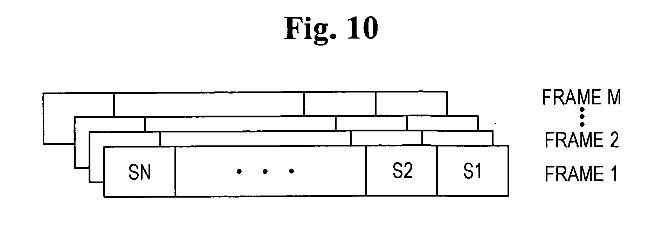

A configuration of the memories 23~23(N) will be described in more detail with reference to Fig. 10. The memories 23~23(N) can be embodied in semiconductor memories, hard disk drives or the like. The memories 23~23(N) store output signals of the respective A/D converters 21~21(N) and additional information transmitted from the local processor 25 via the memory controller 22~22(N).

The size of each memory 23~23(N) can be represented as follows:

$$\text{Memory size} = N_{fr} \text{ X } N_{sl} \text{ X } (F_s \text{ X } 2 \text{ X } z_{max}/c) \qquad \text{Equation 1,}$$

wherein $N_{fr}$ is the number of frames to be stored in the memory, $N_{sl}$ is the number of scan lines to be stored for each frame, $F_s$ is an A/D conversion rate or a sampling frequency, $z_{max}$ is a maximum image depth, and c is a speed of the ultrasound in a human body.

Among the various memories 23~23(N), the memory 23, for example, is divided into frame regions (frame 1 ⋯ frame M) to store data of respective frames, wherein each frame region is divided into a number of scan line regions (S1 ⋯ SN) according to the number of scan lines. The reception signals, converted in the A/D converter 21, are inputted to the memory 23 through the memory controller 22 and the reception signals constructing one frame are stored in the respective memory regions by scan lines. The reception data stored in the memory 23 as above is utilized later when reconstructing an image. More specifically, the memories 23~23(N) store, out of the output signals of the A/D converters 21~21(N), image signals corresponding to at least one frame of the image. The memories 23~23(N) then provide data, the image signals, related to a selected portion of the image to the memory controllers 22~22(N) repeatedly for a specified number of frames.

The local processor 25 receives transfers of reception signals and additional information stored in the memories 23~23(N) by controlling the memory controllers 22~22(N). The local processor 25 generates a control signal based on the transferred reception signals and additional information to control the focusing unit 24. The control signal is generated with inferring optimum values for various parameters, with which an optimum ultrasound image can be obtained by analyzing a sound wave speed, an attenuation, a spectrum analysis of the reception signals, a phase aberration error, and the like. Further, the local processor 25 transmits the reception signals and additional information stored in the memories 23~23(N) to the host processor 300. The local processor 25 receives additional information from the host processor 300 and transmits it to the memories 23~23(N) via the memory controllers 22~22(N). In addition, based on a control signal inputted from the host processor 300, the local processor 25 controls the memory controllers 22~22(N) and the focusing unit 24.

The host processor 300 generates the control signal based on the reception signals and additional information inputted from the local processor 25. The control signal is generated with inferring optimum values for various parameters, with which an optimum ultrasound image can be obtained by analyzing a sound wave speed, an attenuation, a spectrum analysis of the reception signals, a phase aberration error and the like. The ultrasound system is controlled by the control signal generated by the host processor 300 or the local processor 25. Alternatively, it may be possible that only one of the host processor 300 and the local processor 25 generates the control signal.

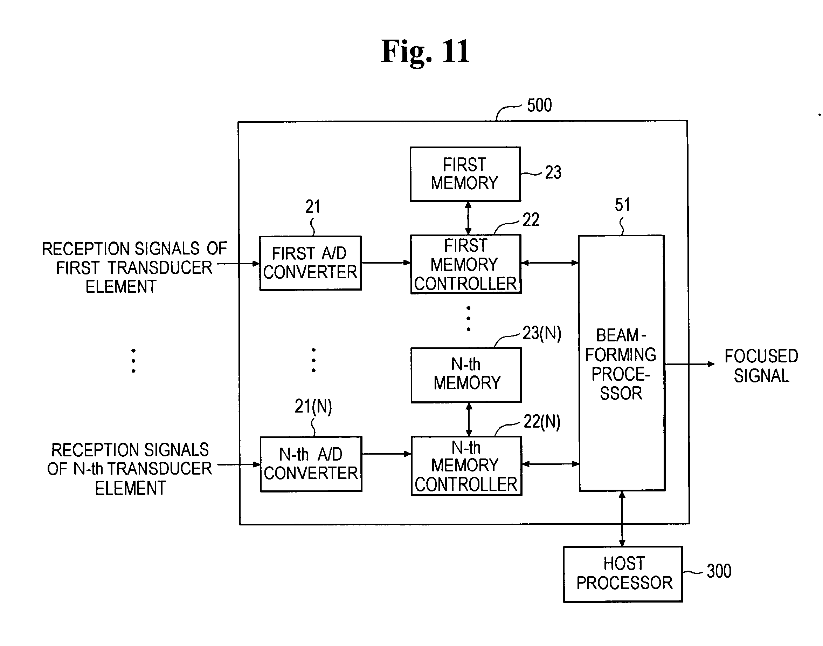

Fig. 11 shows a block diagram of a receive-focusing unit 500 constructed in accordance with another embodiment of the present invention. The receive-focusing unit 500 generally includes a beam-forming processor 51, instead of the focusing unit 24 and the local processor 25 of the receive-focusing unit 200 shown in Fig. 8. The beam-forming processor 51 undertakes all the functions of the focusing unit 24 and the local processor 25 in Fig. 8. That is, the beam-forming processor 51 receives the reception signals and additional information stored in the memories 23~23(N) through the memory controllers 22~22(N) and performs the focusing. It then analyzes the signals inputted through the memory controllers 22~22(N) and generates a control signal to obtain an optimal ultrasound image based on the analysis result. The host processor 300 is coupled with the beam-forming processor 51. Other functional units in the receive-focusing unit 500 have same functions and same connection configuration with those in the receive-focusing unit 200 in Fig. 8. Therefore, they will not be discussed in detail.

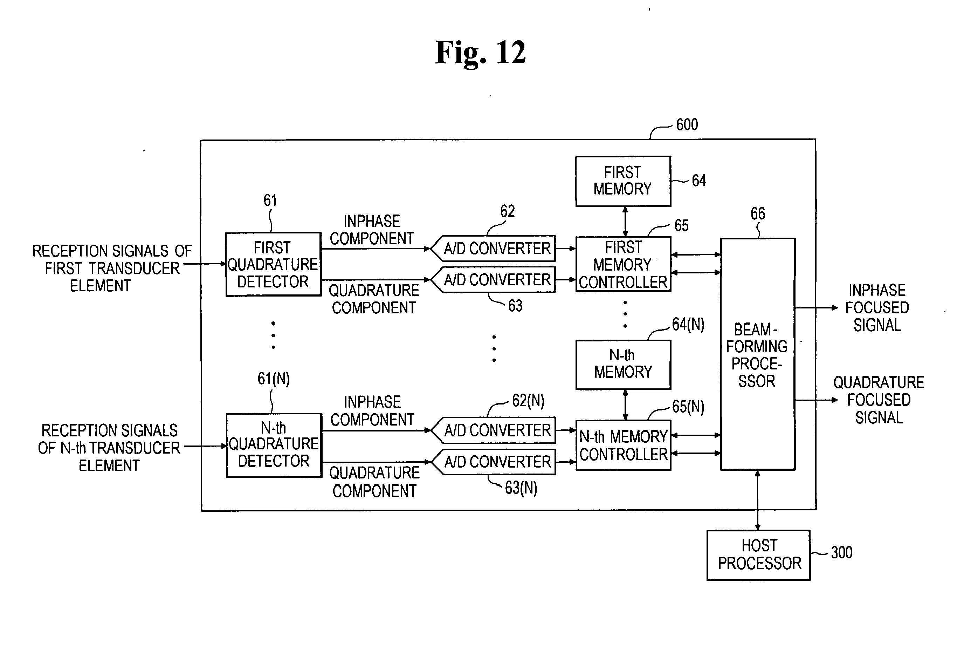

Fig. 12 shows a block diagram of a receive-focusing unit 600 constructed in accordance with still yet another embodiment of the present invention. In addition to the configuration of the receive-focusing unit 500 shown in Fig. 9, the receive-focusing unit 600 further includes quadrature detectors 61~61(N) for dividing the reception signals inputted from the N number of transducers into inphase components and quadrature components. In contrast to the A/D converters 21~21(N) of the receive-focusing unit 500, respective A/D converters 62~62(N) and 63~63(N) of the receive-focusing unit 600 transduce the inphase component reception signals and the quadrature component reception signals, and provide them to memory controllers 65~65(N). Other functions of the memory controllers 65~65(N), the beam-forming processor 66, and the like are identical to those in the aforementioned receive-focusing unit 500. Therefore, they will not be described in detail.

The ultrasound systems shown in Figs. 7 to 12 can reconstruct an image based on additional information, thereby improving the quality of the image. In particular, they can utilize all of the RF reception signals over several frames. In addition, with respect to an image for an organ portion being overlapped between frames, they can improve SNR (signal to noise ratio) by forming the image by overlapping the RF data over several frames. Further, in case a probe moves to repeatedly scan a same region, the RF reception signals can be overlapped even though there is a considerable time gap.

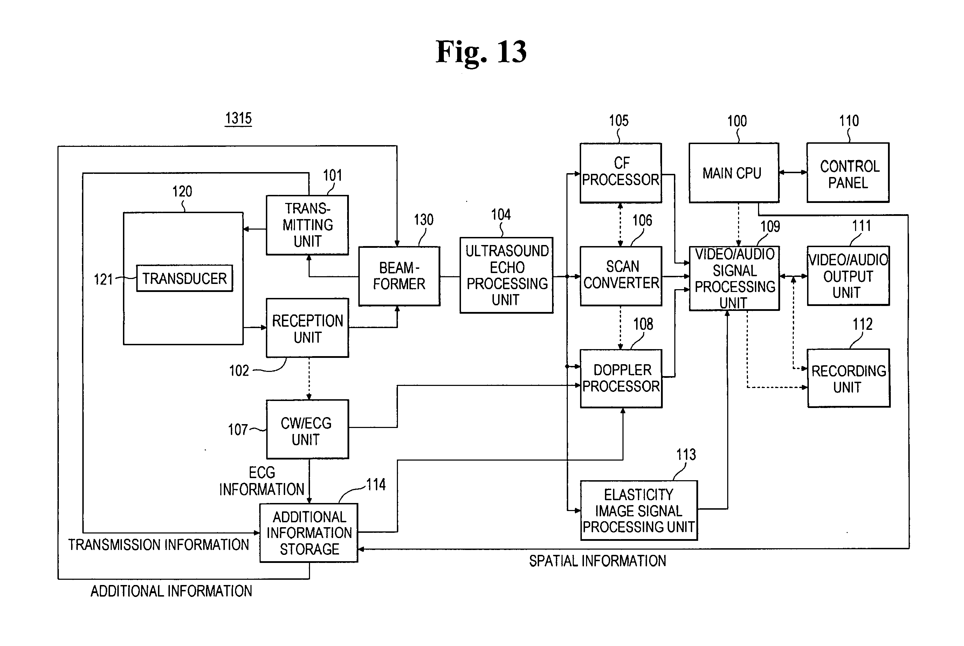

In contrast to the aforementioned first and second preferred embodiments of the present invention, the probe of an ultrasound system 1315 of the present invention may not include a transducer information collecting unit, as shown in Fig. 13. Hereinafter, configurations and functions, which are different from those of the ultrasound system 315 in Fig. 3, will be described. The main CPU 100 of the ultrasound system 1315 generates, in anticipation, spatial information of transducers in accordance with the movement tendency of the probe. Here, the spatial information of the transducers can be generated based on the information set or inputted by a system designer. The spatial information generated by the main CPU 100 is stored in the additional information recording unit 114 and is utilized for constructing or reconstructing a 2-D or 3-D image.

The digital ultrasound system constructed in accordance with the present invention can obtain an ultrasound image having remarkably improved resolution and SNR by constructing or reconstructing a 2-D or 3-D ultrasound image through the use of additional information.

While the present invention has been shown and described with respect to a preferred embodiment, those skilled in the art will recognize that various changes and modifications may be made without departing from the spirit and scope of the invention as defined in the appended claims.

**Claims**

1. An ultrasound system, comprising:

  a probe including a number of transducers for transmitting ultrasound transmission signals to a target object, receiving echo signals reflected from the target object and converting the echo signals into electrical signals;

an analog-to-digital conversion unit for converting the electrical signals into digital data;
a transducer information collecting unit for collecting information on spatial states of the transducers when receiving the echo signals;
a beam-former for forming reception beams based on the converted digital data and the collected information; and
an ultrasound image processing unit for reconstructing an ultrasound image based on the reception beams.

2. An ultrasound system, comprising:

a probe including a number of transducers for transmitting ultrasound transmission signals to a target object, receiving echo signals reflected from the target object and converting the echo signals into electrical signals;
an analog-to-digital conversion unit for converting the electrical signals into digital data;
a spatial information generating unit for generating spatial information on the transducers according to a movement tendency of the probe, said generated spatial information being changed in time;
a beam-former for forming reception beams based on the converted digital data and the change in the generated spatial information; and
an ultrasound image processing unit for reconstructing an ultrasound image based on the reception beams.

3. The system of Claim 1, further comprising:

an ultrasound transmission beam-forming unit for forming the ultrasound transmission signals based on predetermined transmission information, wherein the beam-former includes means for forming the reception beams based on the converted digital data, the collected information and the transmission information.

4. The system of Claim 2, further comprising:

an ultrasound transmission beam-forming unit for forming the ultrasound transmission signals based on predetermined transmission information, wherein the beam-former includes means for forming the reception beams based on the converted digital data, the change in the generated spatial information and the transmission information.

5. The system of Claim 3, further comprising:

a bio-information generating unit for generating bio-information by analyzing the electrical signals, wherein the beam-former includes means for forming the reception beams either based on the converted digital data, the collected information and the bio-information or based on the converted digital data, the collected information, the transmission information and the bio-information.

6. The system of Claim 4, further comprising:

a bio-information generating unit for generating bio-information by analyzing the electrical signals, wherein the beam-former includes means for forming the reception beams either based on the converted digital data, the change in the generated information and the bio-information or based on the converted digital data, the change in the generated information, the transmission information and the bio-information.

7. The system of Claim 3, wherein the ultrasound image processing unit includes means for reconstructing a 2-D or 3-D image.

8. The system of Claim 4, wherein the ultrasound image processing unit includes means for reconstructing a 2-D or 3-D image.

9. An ultrasound system for reconstructing an ultrasound image corresponding to a portion selected by a user out of a preformed ultrasound image, the system comprising:

a probe including a number of transducers for transmitting ultrasound transmission signals to a target object, receiving echo signals reflected from the target object and converting the echo signals into electrical signals;
a transducer information collecting unit for collecting information on spatial states of the transducers when receiving the echo signals;
a receive-focusing unit including an analog-to-digital conversion unit for converting the electrical signals into digital data and a memory for storing the converted digital data to accumulate at least one frame of data for at least a part of the target object therein, the receive-focusing unit being configured to focus the converted digital data in consideration of the collected information, said receive-focusing unit being operable to retrieve and focus the stored digital data corresponding to the selected portion in consideration of the collected information; and an ultrasound image processing unit for forming an ultrasound image based on the focused digital data.

10. The ultrasound system of Claim 9, further comprising:

an ultrasound transmission beam-forming unit for forming the ultrasound transmission signals based on predetermined transmission information, wherein the receive-focusing unit includes means for focusing the converted digital data corresponding to the selected portion in consideration of the collected information and the transmission information.

11. The ultrasound system of Claim 9, further comprising:

a bio-information generating unit for generating bio-information by analyzing the electrical signals, wherein the receive-focusing unit includes means for focusing the converted digital data corresponding to the selected portion in consideration of the collected information and the bio-information or in consideration of the collected information, the transmission information and the bio-information.

12. The ultrasound system of Claim 10, further comprising:

a bio-information generating unit for generating bio-information by analyzing the electrical signals, wherein the receive-focusing unit includes means for focusing the converted digital data corresponding to the selected portion in consideration of the collected information and the bio-information or in consideration of the collected information, the transmission information and the bio-information.

13. The ultrasound system of Claim 11, wherein the ultrasound image processing unit includes means for reconstructing a 2-D or 3-D image.

14. The ultrasound system of Claim 12, wherein the ultrasound image processing unit includes means for reconstructing a 2-D or 3-D image.

# Fig. 1

115

TRANS-MITTING UNIT 101

PROBE

RECEPTION UNIT 102

RECEPTION FOCUSING UNIT 103

ULTRASOUND ECHO PRO-CESSING UNIT 104

CF PROCESSOR 105

MAIN CPU 100

CONTROL PANEL 110

SCAN CONVERTER 106

VIDEO/ AUDIO SIGNAL PROCESSING UNIT 109

VIDEO/AUDIO OUTPUT UNIT 111

CW/ ECG UNIT 107

DOPPLER PROCESSOR 108

RECORDING UNIT 112

EP 1 744 180 A1

# Fig. 2

# Fig. 3

# Fig. 4A

MOVEMENT

# Fig. 4B

MOVE MENT

IMPROVEMENT OF
RESOLUTION

# Fig. 4C

ENLARGEMENT OF VIEW

# Fig. 5A

MOVEMENT

IMPROVEMENT OF
RESOLUTION

# Fig. 5B

IMPROVEMENT OF
RESOLUTION

## Fig. 6A

## Fig. 6B

## Fig. 6C

# Fig. 7

RECEPTION SIGNALS OF FIRST TRANSDUCER ELEMENT → FIRST A/D CONVERTER (21) → FIRST MEMORY CONTROLLER (22) ↔ FIRST MEMORY (23)

RECEPTION SIGNALS OF N-th TRANSDUCER ELEMENT → N-th A/D CONVERTER (21(N)) → N-th MEMORY CONTROLLER (22(N)) ↔ N-th MEMORY (23(N))

FIRST MEMORY CONTROLLER (22) → FOCUSING UNIT (24) → FOCUSED SIGNAL

N-th MEMORY CONTROLLER (22(N)) → FOCUSING UNIT (24)

LOCAL PROCESSOR (25)

HOST PROCESSOR (300)

200

EP 1 744 180 A1

16

# Fig. 8

RECEPTION SIGNALS OF FIRST TRANSDUCER ELEMENT

RECEPTION SIGNALS OF N-th TRANSDUCER ELEMENT

FIRST A/D CONVERTER — 21

N-th A/D CONVERTER — 21(N)

FIRST MEMORY — 23

FIRST MEMORY CONTROLLER — 22

N-th MEMORY — 23(N)

N-th MEMORY CONTROLLER — 22(N)

FOCUSING UNIT — 24

FOCUSED SIGNAL

LOCAL PROCESSOR — 25

HOST PROCESSOR — 300

— 200

EP 1 744 180 A1

# Fig. 9

- MEMORY CONTROL CIRCUIT — 32
- MEMORY CONTROL SIGNAL
- 33
- MEMORY ADDRESS
- DATA FROM A/D CONVERTER
- 34
- TOWARD FOCUSING UNIT
- 31
- EXTERNAL CONNECTION/ CONTROL CIRCUIT
- 35
- TOWARD MEMORY
- 36
- ADDRESS BUS
- DATA BUS
- LOCAL PROCESSOR — 25
- 22

EP 1 744 180 A1

# Fig. 10

| | | | | | FRAME M |
| SN | · · · | | S2 | S1 | FRAME 2 |
| | | | | | FRAME 1 |

# Fig. 11

RECEPTION SIGNALS OF FIRST TRANSDUCER ELEMENT

RECEPTION SIGNALS OF N-th TRANSDUCER ELEMENT

FIRST A/D CONVERTER — 21

N-th A/D CONVERTER — 21(N)

FIRST MEMORY — 23

FIRST MEMORY CONTROLLER — 22

N-th MEMORY — 23(N)

N-th MEMORY CONTROLLER — 22(N)

BEAM-FORMING PROCESSOR — 51

500

FOCUSED SIGNAL

HOST PROCESSOR — 300

# Fig. 12

# Fig. 13

## EUROPEAN SEARCH REPORT

**European Patent Office**

Application Number

EP 06 00 3001

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 538 004 A (BAMBER JEFFREY C [US]) 23 July 1996 (1996-07-23) * abstract; figures 1-5,10A-11 * * column 1, line 6 - column 2, line 6 * * column 2, line 49 - column 8, line 17 * * column 10, line 37 - column 15, line 27 * | 1-14 | INV. G01S15/89 G01S7/52 |
| X | US 2004/006272 A1 (VORTMAN KOBI [IL] ET AL) 8 January 2004 (2004-01-08) * abstract; figures 1,7,8 * * paragraphs [0032] - [0038] * * paragraphs [0083] - [0085] * | 1-8 | |
| X | TAVH B ET AL: "An efficient motion estimation technique for ultrasonic subaperture imaging" ENGINEERING IN MEDICINE AND BIOLOGY SOCIETY, 1998. PROCEEDINGS OF THE 20TH ANNUAL INTERNATIONAL CONFERENCE OF THE IEEE HONG KONG, CHINA 29 OCT.-1 NOV. 1998, PISCATAWAY, NJ, USA,IEEE, US, vol. 2, 29 October 1998 (1998-10-29), pages 816-819, XP010320558 ISBN: 0-7803-5164-9 * the whole document * | 1-8 | TECHNICAL FIELDS SEARCHED (IPC) G01S |
| X | US 2002/023497 A1 (HAYASHI TATSUO [JP] ET AL) 28 February 2002 (2002-02-28) * abstract; figures 1(A)-6(B) * * paragraphs [0002], [0003], [0006], [0007] * * paragraphs [0011] - [0016] * * paragraphs [0026] - [0033] * | 1-8 | |

-/--

The present search report has been drawn up for all claims

| Place of search | Date of completion of the search | Examiner |
|---|---|---|
| Munich | 19 October 2006 | Zaneboni, Thomas |

EPO FORM 1503 03.82 (P04C01)

**European Patent Office**

**EUROPEAN SEARCH REPORT**

Application Number

EP 06 00 3001

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MERCIER L ET AL: "A review of calibration techniques for freehand 3-D ultrasound systems" ULTRASOUND IN MEDICINE AND BIOLOGY, NEW YORK, NY, US, vol. 31, no. 4, April 2005 (2005-04), pages 449-471, XP004849064 ISSN: 0301-5629 * the whole document * | 1-14 | |
| A | US 6 254 539 B1 (PANG LINYONG [US] ET AL) 3 July 2001 (2001-07-03) * the whole document * | 1-14 | |
| A | WO 03/047433 A2 (KONINKL PHILIPS ELECTRONICS NV [NL]) 12 June 2003 (2003-06-12) * the whole document * | 1-14 | |
|  |  |  | TECHNICAL FIELDS SEARCHED (IPC) |

The present search report has been drawn up for all claims

| Place of search | Date of completion of the search | Examiner |
|---|---|---|
| Munich | 19 October 2006 | Zaneboni, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 00 3001

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2006

| Patent document cited in search report | | Publication date | Patent family member(s) | | Publication date |
|---|---|---|---|---|---|
| US 5538004 | A | 23-07-1996 | NONE | | |
| US 2004006272 | A1 | 08-01-2004 | NONE | | |
| US 2002023497 | A1 | 28-02-2002 | GB 2370355 A | | 26-06-2002 |
| | | | JP 2002071790 A | | 12-03-2002 |
| US 6254539 | B1 | 03-07-2001 | NONE | | |
| WO 03047433 | A2 | 12-06-2003 | AU 2002365842 A1 | | 17-06-2003 |
| | | | CN 1599578 A | | 23-03-2005 |
| | | | EP 1476079 A2 | | 17-11-2004 |
| | | | JP 2005511129 T | | 28-04-2005 |
| | | | US 2003105401 A1 | | 05-06-2003 |

EPO FORM P0459

For more details about this annex : see Official Journal of the European Patent Office, No. 12/82